# EUROPÄISCHE PATENTANMELDUNG

(11) **EP 0 878 728 A1**
(43) Veröffentlichungstag der Anmeldung: **18.11.1998**
(21) Anmeldenummer: 98105267.3
(22) Anmeldetag: 24.03.1998
(51) Int. Cl.: G02C 5/20, A61F 9/02, G02C 1/04, G02C 5/14, E04B 1/68, G02C 7/02

(54) **Schutzbrille, insbesondere Arbeitsschutzbrille**

(30) Priorität: 17.05.1997 DE 19720907
(71) Anmelder: Uvex Arbeitsschutz GmbH, 90766 Fürth (DE)
(72) Erfinder: Wiedner, Klaus, 90766 Fürth/Bay (DE); Nussbickl, Herbert, 90425 Nürnberg (DE); Hegendörfer, Erich, 90556 Cadolzburg (DE)
(74) Vertreter: Schneck, Herbert, Dipl.-Phys., Dr.

(57) **Zusammenfassung**

Bei einer Schutzbrille, insbesondere Arbeitsschutzbrille, mit einer einstückigen Scheibe (1), wobei die Scheibe (1) seitliche, sich auf den Kopf des Benutzers zu erstreckende, transparente mit der Scheibe einstückige Ansätze (37) aufweist, ist zur Erweiterung des Gesichtsfeldes und zur Erhöhung der Schutzwirkung zur Seite hin vorgesehen, daß die Scheibe (1) an ihren seitlichen äußeren Enden abgerundet ausgebildet ist und die seitlichen Ansätze (37) eine dieser abgerundeten Konfiguration folgende, konvex nach außen gewölbte Krümmung aufweisen.

## Beschreibung

Die Erfindung richtet sich auf eine Schutzbrille, insbesondere Arbeitsschutzbrille mit einer einstückigen Scheibe, wobei die Scheibe seitliche, sich auf den Kopf des Benutzers zu erstreckende, transparente, mit der Scheibe einstückige Ansätze aufweist. Eine derartige Schutzbrille ist beispielsweise aus DE-U-89 02 696 bekannt.

Bei herkömmlichen derartigen Schutzbrillen gehen die seitlichen Ansätze ausgehend von einer im wesentlichen geraden, vertikalen Knicklinie ebenflächig nach hinten. Dies hat den Nachteil daß die Knicklinie im seitlichen Sichtfeld zu liegen kommt und dadurch der Blick zur Seite erheblich behindert wird.

Es sind auch Brillen bekannt, wo die Scheibe insgesamt so stark gekrümmt ist daß sich die seitlichen Scheibenenden um das Gesicht herum bis in die seitliche Schläfenpartie des Benutzers erstrecken. Derartige Scheiben vermeiden zwar einen das Sichtfeld störenden Knick müssen dafür aber einen Krümmungsradius aufweisen, der einen relativ großen maximalen Abstand zum Gesicht bedingt und auch aus optischen Gründen nicht wünschenswert ist.

Hiervon ausgehend liegt der Erfindung die Aufgabe zugrunde, eine Schutzbrille der eingangs genannten Art so auszugestalten, daß bei ungestörtem Blick auch zur Seite hin ein optimaler seitlicher Schutz der Augen erreicht wird.

Diese Aufgabe wird erfindungsgemäß dadurch gelöst, daß die Sichtscheibe an ihren seitlichen äußeren Enden abgerundet ausgebildet ist und die seitlichen Ansätze eine dieser abgerundeten Konfiguration folgende, konvex nach außen gewölbte Krümmung aufweisen.

Das erfindungsgemäße Konzept weicht von herkömmlichen Lösungen ab und nimmt bewußt eine etwas aufwendigere Formgebung der Spritzgießform in Kauf, um hierdurch zu erreichen, daß in den seitlich auslaufenden Endbereichen der Scheibe Knicklinien vermieden werden. Gleichzeitig wird dadurch aber auch erreicht, daß die seitlichen Ansätze aufgrund ihrer Wölbung nach außen eine erhebliche statische Stabilisierung erfahren und seitlich auftreffende Partikel abgelenkt werden. Darüber hinaus ist es möglich, die hinteren Randbereiche im Schläfenbereich des Benutzers der Gesichtskontur optimal anzupassen.

Vorzugsweise liegt der Krümmungsradius der seitlichen Ansätze zwischen 1 cm und 3 cm, insbesondere beträgt er günstigerweise rund 2 cm. Soweit im Vorstehenden von einem Krümmungsradius die Rede ist, ist dies nicht so zu verstehen, da die seitlichen Ansätze durchgehend den gleichen Krümmungsradius aufweisen müßten, sondern die seitlichen Ansätze können durchaus nicht-sphärisch gekrümmt sein, wobei die genannten Krümmungsradien lediglich das Krümmungsverhalten annähernd wiedergeben.

In weiterer Ausgestaltung der Erfindung ist vorgesehen, daß auf die Oberseite der Sichtscheibe ein Rahmenteil aufrastbar ist, an dessen seitlichen äußeren Enden Bügel anlenkbar sind. Insoweit ist vorzugsweise vorgesehen, daß in die seitlichen äußeren Enden des Rahmenteils Rastteile einrastbar sind, welche Scharnierteile zum Anlenken eines Bügels aufweisen.

Ein bügelseitiges Gelenkteil kann mit einem Kupplungsabschnitt versehen sein, der in ein Kupplungsteil mit Bügel einrastbar ist.

Durch diese vorstehend beschriebene modulare Bauweise ist es möglich, die einzelnen Teile je nach spezifischen Anforderungen auszuwählen, insbesondere unterschiedliche Bügel vorzusehen, und die Beschußfestigkeit zu verbessern.

Die Kupplungsteile mit Bügel sind günstigerweise aus einem weicheren Kunststoff ausgebildet als die übrigen Brillenteile, so daß ein hoher Tragekomfort erreicht wird. Gleichzeitig wird durch diese Weichheit auch eine höhere Elastizität erzielt, so daß vorgesehen sein kann, daß die Kupplungsteile mit Bügel einen Längsschlitz mit Rastausnehmungen zur Längenverstellung aufweisen, in welche ein Rastvorsprung an der Innenseite des Kupplungsteils eingreift.

Die äußeren Enden des Rähmenteils können das Rastteil gabelartig umgreifen, wobei an der Stirnseite des Rastteils und am Boden des Gabelabschnitts der seitlichen Enden Rastvorsprünge bzw. Rastnuten für die Inklinationsverstellung ausgebildet sind.

Der Gabelabschnitt der seitlichen Ansätze kann eine als Anschlag wirkende obere Abdeckung aufweisen.

Das Schwenklager für die Bügel kann günstigerweise so realisiert werden, daß in einem der Gabelabschnitte eine Schwenklagerausnehmung und an der einen Seite jedes Rastteils eine in die Schwenklagerausnehmung einrastbarer Lagerzapfen ausgebildet ist, der zur Erleichterung des Einrastvorgangs in Einschubrichtung angeschrägt sein kann.

Das obere Rahmenteil kann günstigerweise ein sich nach hinten auf das Gesicht des Benutzers zu erstreckenden Abdeckungsabschnitt aufweisen, der eine formschlüssige Anlage an der Stirn ermöglicht und dementsprechend das Eindringen von Tropfen und Partikeln von der Oberseite her verhindert.

Das obere Rahmenteil kann an seiner Unterseite eine Nut zum Einsetzen des Oberrandes der Scheibe aufweisen. Die Scheibe kann jeweils an ihrer seitlichen Außenseite Ausnehmungen besitzen, und das obere Rahmenteil mit seitlichen korrespondierenden Vorsprüngen zum Festlegen der Scheibe versehen sein.

Günstigerweise sind die Vorsprünge am oberen Rahmenteil vertieft angeordnet, so daß bei eingesetzter Scheibe ein innerer Begrenzungswulst der den oberen Rand der Scheibe aufnehmenden Nut den Verriegelungsbereich übergreift. Es wird also eine Art Bajonettverschluß zur Festlegung der Scheibe geschaffen.

Im Bereich der Nasenbrücke der Scheibe kann ein Drahtabschnitt eingespritzt sein, dessen seitliche äußere Enden sich beiderseits der Nase nach außen erstrecken, wobei auf diese Enden Nasenpads oder die Endabschnitte einer Nasenschlinge aufgesetzt sind. Eine solche Ausgestaltung ist von Sonnenbrillen oder optischen Brillen her an sich bekannt, wurde aber in Verbindung mit einteiligen Arbeitsschutzbrillen bisher noch nicht realisiert. Hierdurch wird ein besonders hoher Tragekomfort erreicht.

Die Scheibe ist vorzugsweise sphärisch gekrümmt und weist deshalb günstige optische Eigenschaften auf.

An den Bügelenden können Ausnehmungen für ein Halteband ausgebildet sein.

Bei einer Variante kann vorgesehen sein, daß an den Rahmen ein Nasenteil angespritzt ist.

Eine weitere Variante sieht vor, daß die Scheibe sich nach hinten erstrekkende, plattenartige Ansätze aufweist, in welchen schräg zur Horizontalen verlaufende langlöcher ausgebildet sind, wobei an der Innenseite des Rahmens korrespondierende Verriegelungsansätze an Haltestielen ausgebildet sind, welche in die Langlöcher einsetzbar und durch Verschwenken des Rahmens festlegbar sind, wobei die Scheibe in Mitte einen Rastvorsprung und der Rahmen eine Rastausnehmung aufweisen.

Nachfolgend wird die Erfindung anhand bevorzugter Ausführungsbeispiele in Verbindung mit der Zeichnung näher erläutert. Dabei Zeigen:
- Fig. 1: eine Explosionsdarstellung einer erfindungsgemäßen Brille,
- Fig. 2: eine Ansicht des Rahmens von innen,
- Fig. 3: einen Längsschnitt durch ein Gelenkteil in vergrößertem Maßstab,
- Fig. 4: eine vergrößerte Darstellung eines Rastteils von innen,
- Fig. 5: eine gegenüber Fig. 4 um 90 ° versetzte Ansicht des Rastteils,
- Fig. 6: eine Ansicht des Kupplungsteils mit Bügel,

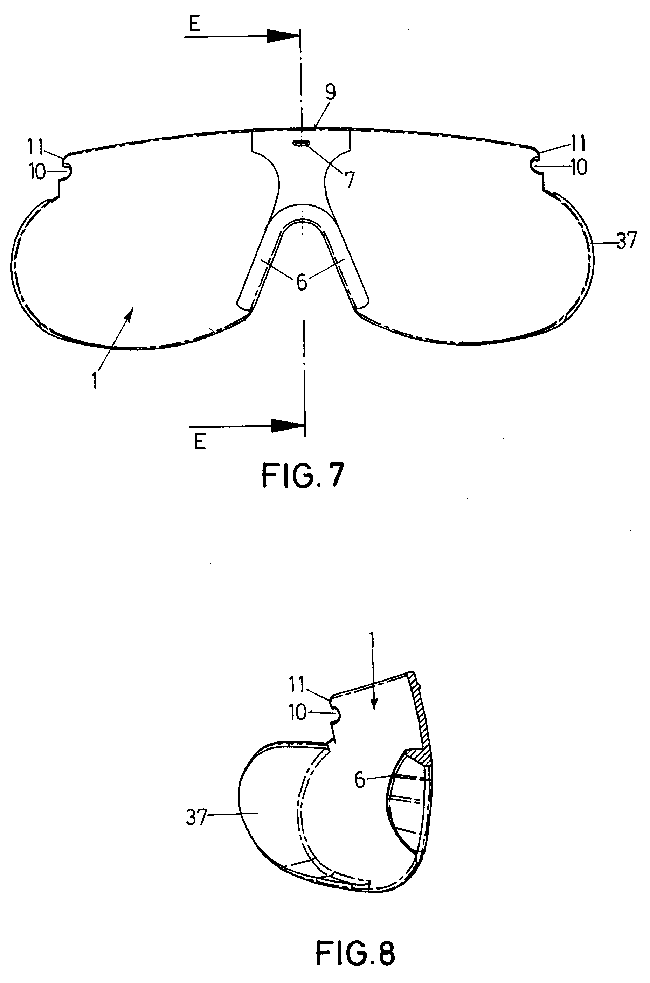

- Fig. 7: eine Ansicht der Scheibe ohne Rahmen,
- Fig. 8: einen Schnitt längs der Linie E-E in Fig. 7,
- Fig. 9: eine perspektivische Ansicht einer abgewandelten Ausführungsform des Rahmens,
- Fig. 10: eine perspektivische Ansicht der Brille und des Rahmens gemäß einer weiteren Ausführungsform,
- Fig. 11: eine Darstellung des Nasenbereichs mit eingespritzten Drahtabschnitten,

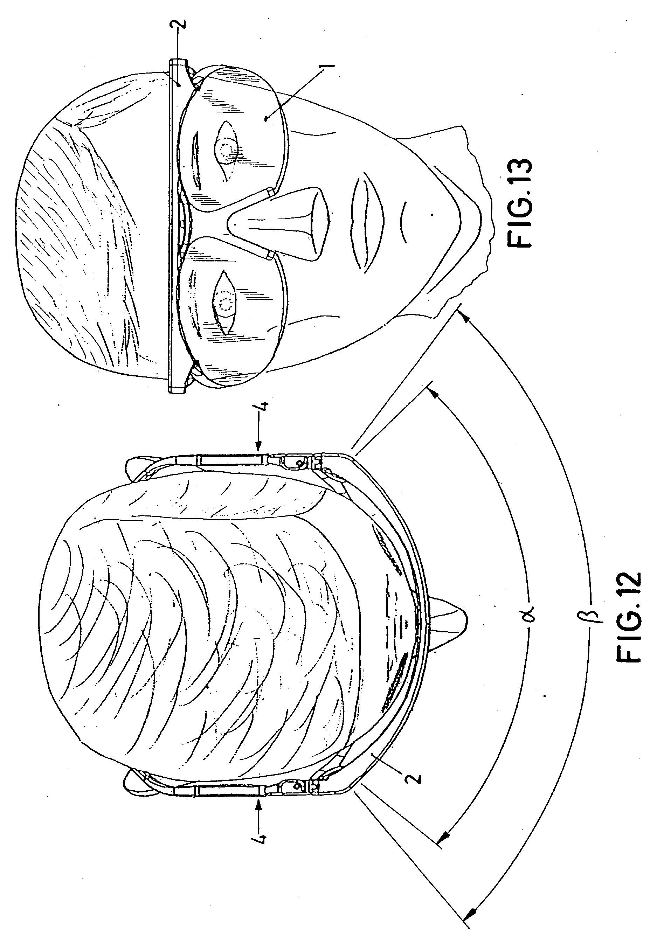

- Fig. 12: eine Aufsicht auf die Brille im getragenen Zustand und
- Fig. 13: eine Ansicht der Brille im getragenen Zustand von vorne.

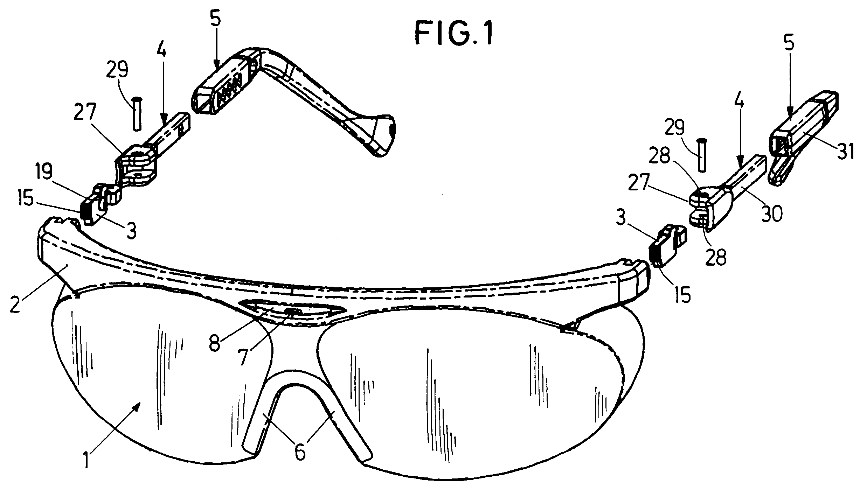

Eine in Fig. 1 dargestellte Brille umfaßt eine Scheibe 1, einen Rahmen 2, beiderseits mit dem Rahmen um eine horizontale Achse schwenkbar verbindbare Rastteile 3, mit diesen um eine vertikale Achse schwenkbar verbindbare Gelenkteile 4 und auf die Gelenkteile 4 aufrastbare Kupplungsteile mit Bügel 5.

Die Scheibe 1 ist einstückig ausgebildet und bei der in Fig. 1 dargestellten Ausführungsform ist an die Scheibe eine Nasenauflage 6 angespritzt.

Der Rahmen 2 ist auf die Scheibe 1 aufgerastet. Hierzu dient einerseits in der Mitte ein Rastvorsprung 7 an der Scheibe sowie eine als Längsschlitz ausgebildete Rastausnehmung 8 im Rahmen sowie ein Arretierzapfen 9' am Rahmen 2, der die Oberkante 9 der Scheibe 1 übergreift.

Die Scheibe 1 weist an beiden Außenseiten Halteeinschnitte 10 und hierdurch gebildete Vorsprünge 11 auf, welche in eine korrespondierende, bajonettartige Halteanordnung 12 an den Seiten des Rahmens 2 einschiebbar sind, wobei dann die Oberkante 9 der Scheibe 1 in einer Nut 13 an der Unterseite des Rahmens 2 zu liegen kommt. Dabei übergreift die innere Begrenzungswand 14 dieser Nut im Haltebereich 12 die Scheibe, so daß diese nach innen hin im Haltebereich festgelegt ist.

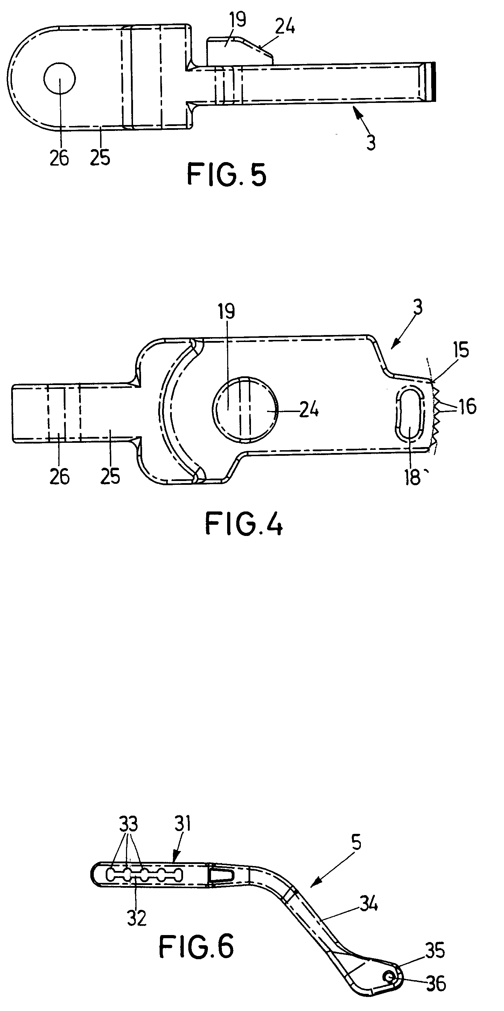

Wie insbesondere aus Fig. 4 und Fig. 5 erkennbar ist, ist die Stirnseite 15 jedes Rastteils 3 mit Rippen 16 versehen, welche mit korrespondierenden Rippen 17 an dem Rahmen 2 unter Ausbildung einer Inklinations-Rastung zusanunenwirken. Eine Ausnehmung 18 im Rastteil 3 sorgt für eine federelastische Einrastung.

An der Innenseite des Rastteils 3 ist ein Schwenklagerbolzen 19 angespritzt, er ist einrastbar in korrespondierende Schwenklagerausnehmungen 20 an sich nach hinten erstreckenden seitlichen Abschnitten 21 des Rahmens 2.

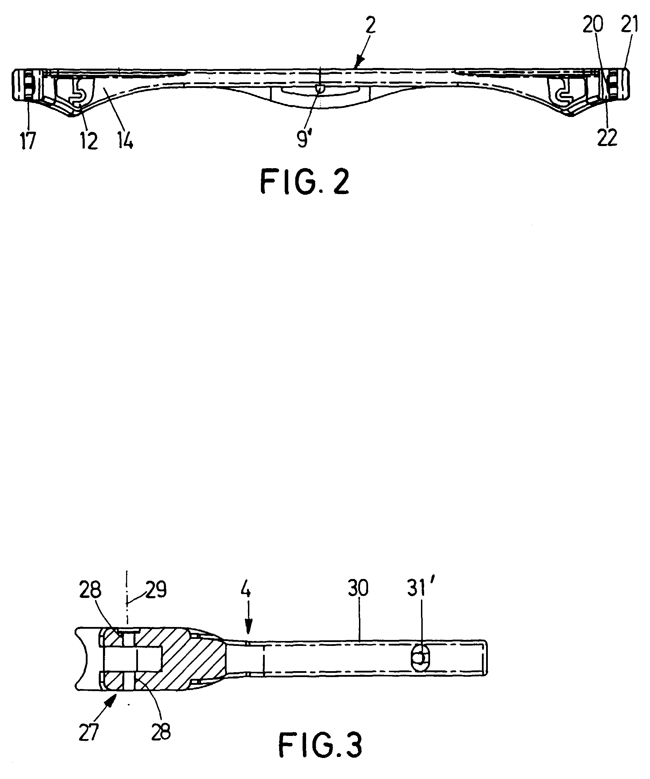

Diese Abschnitte 21 sind im Schnitt, wie aus der Ansicht von hinten gemäß Fig. 2 deutlich wird, gabelartig ausgebildet, wobei die Schwenklagerausnehmung 20 im inneren Gabelabschnitt 22 ausgebildet ist.

Die Gabelabschnitte 21 weisen an ihrer Oberseite eine Abdeckung 23 auf, die verhindert, daß eine Schwenkbewegung über die horizontale Position hinaus möglich ist. Um das Einrasten des Schwenklagerbolzens 19 in die Rastausnehmung 20 zu erleichtern, ist der Schwenklagerbolzen 19 in Einschubrichtung mit einer abgeschrägten Fläche 24 versehen.

Das bezogen auf die Scheibe 1 rückwärtige Ende des Rastteils 3 weist einen Ansatz 25 mit einer Schwenklagerbohrung 26 auf, die, wie nachstehend noch beschrieben, ein Schwenkgelenk um eine vertikale Schwenkachse zum Einklappen der Bügel definiert.

Zur Ausbildung des Schwenkgelenks dient auch das Gelenkteil 4, welches, wie insbesondere aus Fig. 1 deutlich wird, einen gabelartigen Abschnitt 27 mit zwei Schwenklagerbohrungen 28 aufweist, wobei der Gabelabschnitt 27 den Ansatz 25 des Rastteils 3 oben und unten übergreift, so daß die Bohrungen 26 und 28 fluchten und dementsprechend ein Rohrniet 29 als Schwenklagerbolzen einsetzbar ist.

An den Gabelabschnitt 27 des Gelenkteils 4 schließt sich ein Kupplungsabschnitt 30 an, der an seiner Innenseite einen Rastvorsprung 31' aufweist.

Das Kupplungsteil mit Bügel 5 weist einen Hülsenabschnitt 31 auf, in den der Kupplungsabschnitt 30 des Gelenkteils 4 einschiebbar ist. An der Innenseite des Hülsenabschnitts 31 ist ein Längsschlitz 32 mit einer Mehrzahl von Rastausnehmungen 33 vorgesehen, in die der Rastvorsprung 31 einrastbar ist, so daß auf diese Weise eine Längenverstellung realisierbar ist.

An den Hülsenabschnitt 31 schließt sich ein Ohrabschnitt 34 an, der an seinem äußeren Ende 35 eine Bohrung 36 für ein Halteband oder dergleichen aufweist.

Die Scheibe 1 ist mit seitlichen Ansätzen 37 versehen, welche sich vom äußeren Rand 38 der Scheibe 1 nach hinten auf das Gesicht des Benutzers zu erstrecken. Die Kante 38 weist einen relativ engen Krümmungsradius auf bzw. läßt sich - da sie nicht exakt kreisabschnittförmig ausgebildet ist - durch einen solchen Krümmungsradius annähern. Hierdurch entsteht ein Bereich 39, der die effektive Breite der Scheibe 1 vergrößert, so daß der Benutzer ungestört durch vertikal verlaufende Kanten, wie sie aus dem Stand der Technik bekannt sind, frei zur Seite schauen kann.

Die Ansätze 37 weisen eine konvexe Wölbung auf, die dem Verlauf der Kante 38 folgt, so daß eine hohe Stabilität der Ansätze 37 auch gegen von außen aufprallende Partikel erzielt wird.

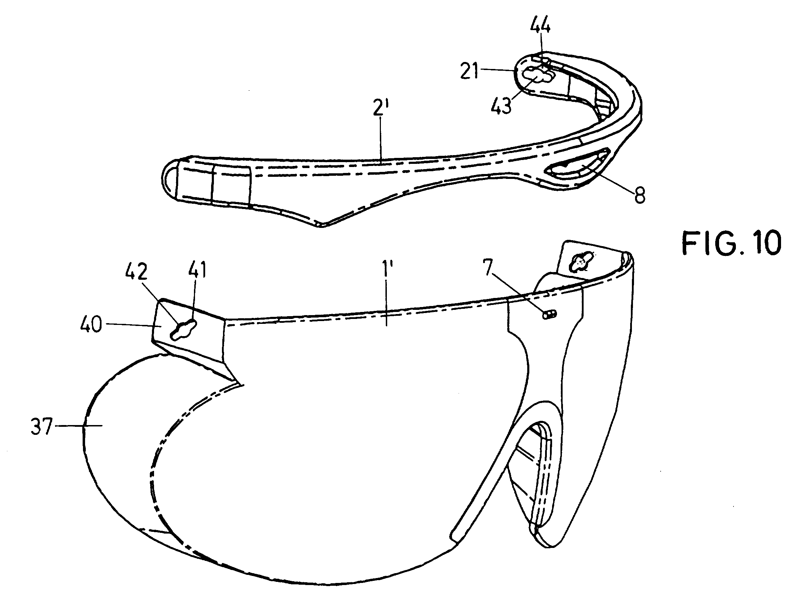

In Fig. 10 ist eine abgewandelte Ausführungsform dargestellt, bei welcher oberhalb der gewölbten seitlichen Ansätze 37 noch plattenartige Ansätze 40 vorgesehen sind, welche ein Langloch 41 mit einer mittigen Verbreiterung 42 aufweisen.

An der Innenseite der äußeren Abschnitte 21 des Rahmenteils 2' ist jeweils ein in der Form korrespondierender Verriegelungsansatz 43 mit einem Haltestiel 44 vorgesehen, welcher horizontal in der Ebene des Rahmenteils 2' verläuft, wohin gegen das Langloch 41 zur Horizontalen eine Neigung von etwa 45 ° aufweist. Dementsprechend wird das Rahmenteil zum Befestigen ebenfalls mit dieser Neigung auf die Oberseite der Scheibe 1' aufgesetzt, so daß die Verriegelungsansätze 43 das Langloch durchsetzen, woraufhin dann die Vorderseite des Rahmens 2' nach unten geschwenkt wird und im Mittelbereich eine Verrastung über den Rastvorsprung 7 und die Rastausnehmung erfolgt. Die äußeren Enden des Rahmens 2' sind dementsprechend formschlüssig festgelegt, weil der Verriegelungsansatz 43 gegen das schräg gestellte Langloch 41 verschwenkt ist.

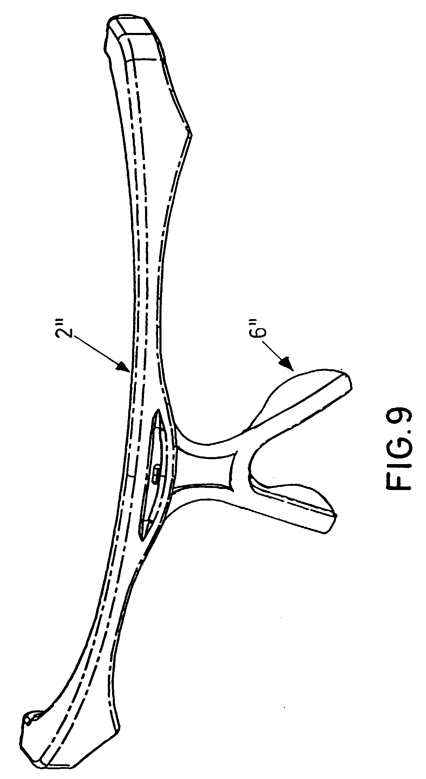

In Fig. 9 ist eine modifizierte Ausführungsform des Rahmenteils 2'' dargestellt, wobei das Nasenauflageteil 6'' mit dem Rahmen einstückig gespritzt ist und dementsprechend mit dem Rahmen 2'' an der Scheibe 1 befestigt wird.

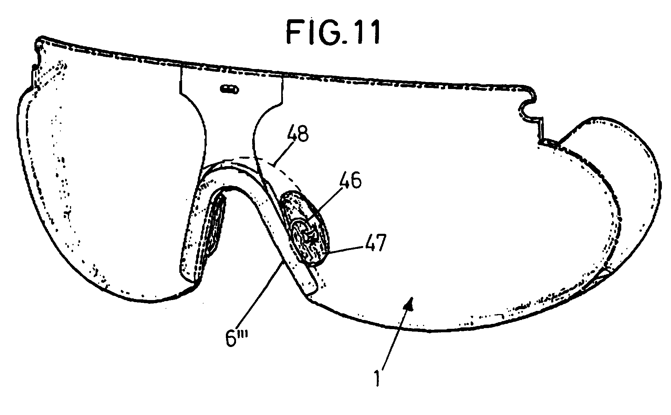

In Fig. 11 ist eine weitere modifizierte Ausführungsform dargestellt. Dabei sind in das Nasenauflageteil 6''' Drähte 46 eingespritzt, an welchen Nasenpads 47 befestigt sind. Solche an Drähten angeordnete Nasenpads 47 sind bei Arbeitsschutzbrille an sich unüblich, ermöglichen jedoch in Kombination mit der einstückigen Scheibe und den sicherheitstechmschen Vorteilen einer solchen Scheibe trotzdem eine optimale Anpassung an die Nasenkonfiguration des Benutzers. Statt eines Nasenpads 47 kann an den Drähten 46, wie bei 48 gestrichelt angedeutet, auch eine Nasenschlinge befestigt sein, d. h. ein durchgehendes Kunststoffteil, welches sich zwischen den Drahtabschnittten über die Nase des Benutzers erstreckt.

## Patentansprüche

1. Schutzbrille, insbesondere Arbeitsschutzbrille, mit einer einstückigen Scheibe (1), wobei die Scheibe (1) seitliche, sich auf den Kopf des Benutzers zu erstreckende, transparente, mit der Scheibe einstückige Ansätze (37) aufweist, **dadurch gekennzeichnet, daß** die Scheibe (1) an ihren seitlichen äußeren Enden abgerundet ausgebildet ist und die seitlichen Ansätze (37) eine dieser abgerundeten Konfiguration folgende, konvex nach außen gewölbte Krümmung aufweisen.

2. Schutzbrille nach Anspruch 1, **dadurch gekennzeichnet, daß** der mittlere Krümmungsradius (R) der seitlichen Ansätze 1 cm bis 3 cm, vorzugsweise etwa 2 cm beträgt.

3. Schutzbrille nach Anspruch 1, **dadurch gekennzeichnet, daß** auf die Oberseite der Scheibe (1) ein Rahmenteil (2) aufrastbar ist, an dessen seitlichen äußeren Abschnitte (21) Bügel (5) anlenkbar sind.

4. Schutzbrille nach Anspruch 3, **dadurch gekennzeichnet, daß** in die seitlichen äußeren Enden des Rahmenteils (2) Rastteile (3) einrastbar sind, welche Scharnierteile (28, 29) zum Anlenken eines Bügels (5)aufweisen.

5. Schutzbrille nach Anspruch 4, **dadurch gekennzeichnet, daß** ein bügelseitige Gelenkteil (4) mit einem Kupplungsabschnitt (30) ausgestaltet ist, der in ein Kupplungsteil mit Bügel (5) einrastbar ist.

6. Schutzbrille nach Anspruch 5, **dadurch gekennzeichnet, daß** das Kupplungsteil mit Bügel (5) aus einem weicheren Kunststoff ausgebildet ist als die übrigen Brillenteile.

7. Schutzbrille nach Anspruch 6, **dadurch gekennzeichnet, daß** das Kupplungsteil mit Bügel (5) einen Längsschlitz (32) mit Rastausnehmungen (33) zur Längenverstellung aufweisen, in welche ein Rastvorsprung (31) an der Innenseite des Gelenkteils (4) eingreift.

8. Schutzbrille nach Anspruch 3, **dadurch gekennzeichnet, daß** die äußeren Enden des Rahmenteils (2) das Rastteil (3) gabelartig umgreifen, wobei an der Stirnseite (15) des Rastteils (3) und am U-Boden des Gabelabschnitts (22) der seitlichen End-Abschnitte (21) Rastvorsprünge (16, 17) für die Inklinationsverstellung ausgebildet sind.

9. Schutzbrille nach Anspruch 8, **dadurch gekennzeichnet, daß** der Gabelabschnitt (22) eine stabilisierende obere Abdeckung (23) aufweist, die das Aushebeln des Rastteils (3) verhindert.

10. Schutzbrille nach Anspruch 8, **dadurch gekennzeichnet, daß** in einem der Gabelabschnitte (22) eine Schwenklagerausnehmung (20) und an der einen Seite jedes Rastteils (3) ein in die Schwenklagerausnehmung (20) einrastbarer Schwenklagerbolzen (19)ausgebildet ist.

11. Schutzbrille nach Anspruch 10, **dadurch gekennzeichnet, daß** der Schwenklagerbolzen (19) in Einschubrichtung eine Abschrägung (24) aufweist.

12. Schutzbrille nach Anspruch 3, **dadurch gekennzeichnet, daß** der Rahmen (2) einen sich nach hinten auf das Gesicht des Benutzers zu erstrekkenden Abdeckungsabschnitt (45) aufweist.

13. Schutzbrille, insbesondere nach Anspruch 3, **dadurch gekennzeichnet, daß** der Rahmen (2) an seiner Unterseite eine Nut (13) zum Einsetzen der Oberkante (9) der Scheibe (1), daß die Scheibe (1) an ihrer seitlichen Außenseite Ausnehmungen (10), und daß der Rahmen (2) seitliche korrespondierende Halteanordnungen (12) zum Festlegen der Scheibe (1) aufweist.

14. Schutzbrille nach Anspruch 13, **dadurch gekennzeichnet, daß** die Halteanordnungen (12) am Rahmen (2) vertieft angeordnet sind, so daß bei eingesetzter Scheibe (1) die innere Begrenuungswand (14) der die Oberkante (9) der Scheibe (1) aufnehmenden Nut (13) den Verriegelungsbereich übergreift.

15. Schutzbrille nach Anspruch 13, **dadurch gekennzeichnet, daß** der Rahmen (2) eine mittige Rastausnehmung (8) für einen an der Außenseite der Scheibe angeordneten Rastvorsprung (7) aufweist.

16. Schutzbrille, insbesondere nach Anspruch 1, **dadurch gekennzeichnet, daß** in die Nasenbrücke ein Drahtabschnitt (46) eingespritzt ist, dessen seitliche äußere Enden sich beidseits der Nase nach außen erstrecken, wobei auf diese Enden Nasenpads (47) oder die Endabschnitte einer Nasenschlinge aufgesetzt sind.

17. Schutzbrille nach Anspruch 1, **dadurch gekennzeichnet, daß** die Scheibe (1) sphärisch gekrümmt ist.

18. Schutzbrille nach Anspruch 1, **dadurch gekennzeichnet, daß** an den Enden (35) des Kupplungsteils mit Bügel (5) Ausnehmungen (36) für ein Halteband ausgebildet sind.

19. Schutzbrille nach Anspruch 1, **dadurch gekennzeichnet, daß** an den Rahmen (2'') ein Nasenteil (6'') angespritzt ist.

20. Schutzbrille nach Anspruch 1, **dadurch gekennzeichnet, daß** die Scheibe nach hinten erstreckende, plattenartige Ansätze (40) aufweist, in welchen schräg zur Horizontalen verlaufende Langlöcher (41) ausgebildet sind, wobei an der Innenseite des Rahmens korrespondierende Verriegelungsansätze (43) an Haltestielen (44) ausgebildet sind, welche in die Langlöcher (41) einsetzbar und durch Verschwenken des Rahmens (2) festlegbar sind, wobei die Scheibe (1') in der Mitte einen Rastvorsprung (7) und der Rahmen (2) eine Rastausnehmung (8) aufweist.

21. Schutzbrille nach Anspruch 1**, dadurch gekennzeichnet, daß** das Gesichtsfeld α 120° bis 130° beträgt.

22. Schutzbrille nach Anspruch 21, **dadurch gekennzeichnet, daß** das optisch korrekte Gesichtsfeld β ca. 110° beträgt.
